# EUROPÄISCHE PATENTSCHRIFT

(11) **EP 0 968 680 B1**
(45) Veröffentlichungstag und Bekanntmachung des Hinweises auf die Patenterteilung: **08.05.2002**
(21) Anmeldenummer: 99111497.6
(22) Anmeldetag: 14.06.1999
(51) Int. Cl.: A61B 5/00, A61B 5/087, A61B 5/08

(54) **Test- und Analysesystem für Atemfunktionen**
System for testing and analysing the respiratory functions
Dispositif destiné à l'essai et à l'analyse des fonctions respiratoires

(30) Priorität: 15.06.1998 DE 19826266
(43) Veröffentlichungstag der Anmeldung: 05.01.2000
(73) Patentinhaber: Vilozni, Daphna, Hod-Hasharon (IL)
(72) Erfinder: Vilozni, Daphna, Hod-Hasharon (IL)
(74) Vertreter: Götz, Georg, Dipl.-Ing.

(56) Entgegenhaltungen:
- WO-A-94/14374
- WO-A-97/01984
- US-A- 3 991 304
- US-A- 5 555 891

## Beschreibung

Die Erfindung betrifft im allgemeinen ein System zum Test und zur Analyse von Lungen- oder sonstigen Atemfunktionen von Personen oder Tieren, insbesondere von Vorschulkindern, mit einem oder mehreren deren Atmung erfassenden Meßmitteln und mit einem auf deren Psyche und/oder Nervensystem einwirkenden Ausgabemedium, das von einer Reiz-, Animations- oder sonstigen Informations-Musterquelle gespeist wird.

Ein solches System wird im Oberbegriff des Anspruchs 1 definiert und entspricht dem bekannten System aus US-A-3 991 304. Darüberhinaus offenbart die Druckschrifft WO 94/14344 eine Atmungsregelvorrichtung, welche aus einem Atmungsmeßmittel, aus einem Steuerelement, aus einer Musterquelle sowie aus einem Ausgabemedium besteht. Die Informationen aus der Musterquelle zu dem Ausgabemedium entstehen aus einem Vergleichsprozess zwischen dem tatsächlich gemessenem Atmungssignal und aus dem Speicher festgelegten Daten, welcher Vergleichprozess in dem Steuerelement stattfindet.

Für Lungenkrankheiten bildet die Atemmessung ein Kernstück der Diagnostik. Sie hat sich bei Kindern ab 6 Jahre und älter bewährt. Allerdings werden dazu an den Patienten hohe Anforderungen gestellt. Er muß lernen, mit maximaler Tiefe einzuatmen und auf Befehl mit höchster Kraft auszuatmen, bis die gesamte Luft ausgeatmet ist. Das dazu bekannte Atmungsmeßsystem ist auf Erwachsene zugeschnitten, wobei die Angst von Kindern nicht berücksichtigt ist.

Vorschulkinder im Alter bis zu 6 Jahren sind sich normalerweise nicht bewußt, daß sie atmen. Bei ihren instinktiven Atmungsvorgängen können sie nicht zwischen Ein- und Ausatmung unterscheiden. Von Vorschulkindem ist es nicht zu erwarten, daß sie auf Anforderung mit maximaler Tiefe einatmen und mit maximaler Kraft ausatmen, bis alle mögliche Luft ausgeatmet ist. Zudem können sie kaum dazu überredet werden, sich freiwillig den Atmungsmeßvorgängen zu fügen und einen beträchtlichen Übungsaufwand auf sich zu nehmen, weil sie für diesen Grad aktiver Kooperation zu jung sind. Außerdem haben Vorschulkinder Angst vor Atmungsmeßgeräten.

Femer ist eine Teilatemmessung bekannt, die auf subjektiver Atemreizung oder - auslösung des Patienten basiert. Der Patient wird mit Reizen überflutet, welche zu einem kräftigen und langen Ausatmen animieren, allerdings ungeachtet des Einatmens vor und nach dem stimulierten Atemmanöver. Dieses Einatmen ist aber kritisch für korrekte Meßwerte. Die Animation basiert auf den Instinkten des Patienten, insbesondere im Kindesalter, tief einzuatmen, bevor kräftig ausgeblasen wird, um zum Beispiel ein Spiel zu gewinnen (z. B.: Ausblasen von Kerzen, Hochspringen usw.). Von solchen Atmungsaktivitäten läßt sich keine Gewißheit darüber gewinnen, daß das Kind vorher maximale Luftmengen eingeatmet hat.

Der Erfindung liegt die Aufgabe zugrunde, Atemtest und -Analysesysteme so weiter zu entwickeln, daß auch Lungenfunktionen und Atmungsaktivitäten von Vorschulkindern zuverlässig gemessen und analysiert werden können.

Zur Lösung wird bei einem Test- und Analysesystem der eingangs genannten Art erfindungsgemäß ein System gemäß Anspruch 1 vorgeschlagen. Bei ihm werden im allgemeinen dem oder den Meßmitteln an deren Ausgängen ein oder mehrere Filter nachgeschaltet die ausgangsseitig mit der Musterquelle zu deren Ansteuerung gekoppelt und zur Weiterverarbeitung von Meßgrößen in Ansteuerungssignale derart ausgebildet sind, daß die Musterquelle auszugebende Informationen in das Ausgabemedium in Abhängigkeit von der Atmung bzw. vom Atmungszustand einspeist

Mit dieser Erfindung läßt sich das Vorschulkind unbewußt dazu bringen, vor einer zu messenden Ausatmung tief einzuatmen, wobei auch die eingeatmete Menge bzw. das eingeatmete Volumen an Luft gemessen wird. Die erfinderische Idee besteht darin, atmende Lebewesen, insbesondere Kinder im Vorschulalter oder behinderte Kinder, durch ein auf sie einwirkendes Ausgabemedium, welches z. B. Animationsgrafiken liefern kann, zum korrekten Lungenfunktionsmanöver anzuleiten. Geatmetes Luftvolumen, Volumenstrom bzw. -durchfluß und Atemstörungen werden dabei von den Meßmitteln registriert und beeinflussen über den oder die Filter das Ausgabemedium, beispielsweise die bewegten Bilder auf einem Bildschirm, die das Vorschulkind während der Messung verfolgt. Die vom Ausgabemedium dem Vorschulkind oder sonstigen Lebewesen dargestellten Muster, beispielsweise bewegte Figuren auf einem Bildschirm, lassen sich in an sich bekannter Weise so ausbilden, daß die Atmung des Kindes positiv beeinflußt wird. Mit besonderem Vorteil werden die vom Ausgabemedium auf das Kind oder sonstige Lebewesen ausgehenden Reize, beispielsweise in Form bewegter Grafik, in eine inhaltlich zusammenhängende Geschichte eingebettet, die durch die Atmung des Kindes über die Meßmittel, die Filterung und ggf. eine Rechnereinheit in ihrem Verlauf interaktiv gesteuert werden. Dabei können die vom Ausgabemedium ausgehenden Geschichten für das Kind auch erzieherische Wirkungen haben.

Ein weiterer, mit der Erfindung erzielter Vorteil besteht darin, daß eine vollständige Atmungsmessung erzielt wird, da ein Erfassen nicht nur des Ausatmens, sondern auch des Einatmens vorgesehen ist. Hier sind die Grundlagen dafür geschaffen, Flutatmung zu analysieren und Bronchien-Stimulationstests durchzuführen.

Zweckmäßig ist dem oder den Filterausgängen eine Rechnereinheit nachgeschaltet, die ein Speichermedium ansteuert, das als Quelle für die dem Ausgabemedium zu liefemden Muster dient. Dabei kann die gespeicherte Information Muster enthalten, welche der Kinderwelt entstammende Anreize oder Animationen bilden. Damit ist es möglich, Vorschulkindern separate Atemschritte beizubringen, welche die gesamte Atmung ausmachen oder verschiedene Stufen eines erzwungenen Ausatmungsmanövers bilden. Die Spiele können zu lösende Aufgaben verschiedener Schwierigkeitsgrade aufweisen, wobei beim Lösen der Aufgabe auch eine Belohnung vorgesehen ist. Die Reizmuster können in Form einer Geschichte angelegt sein, welche dem begrenzten Konzentrationszeitraum des Kindes Rechnung trägt, was das Zusammenwirken mit einer Bronchien-Stimulierung angeht.

Um im Analyse- und Testsystem Angst einflößende Elemente zu reduzieren und die Teilnahme- und Kooperationsbereitschaft der betroffenen Patienten zu erhöhen, ist es im Falle von Vorschulkindern zweckmäßig, die Systemausrüstung (Mundstücke, Lungentachygraphen usw.) an die Größe, Stärke und das Gemüt von Vorschulkindern anzupassen.

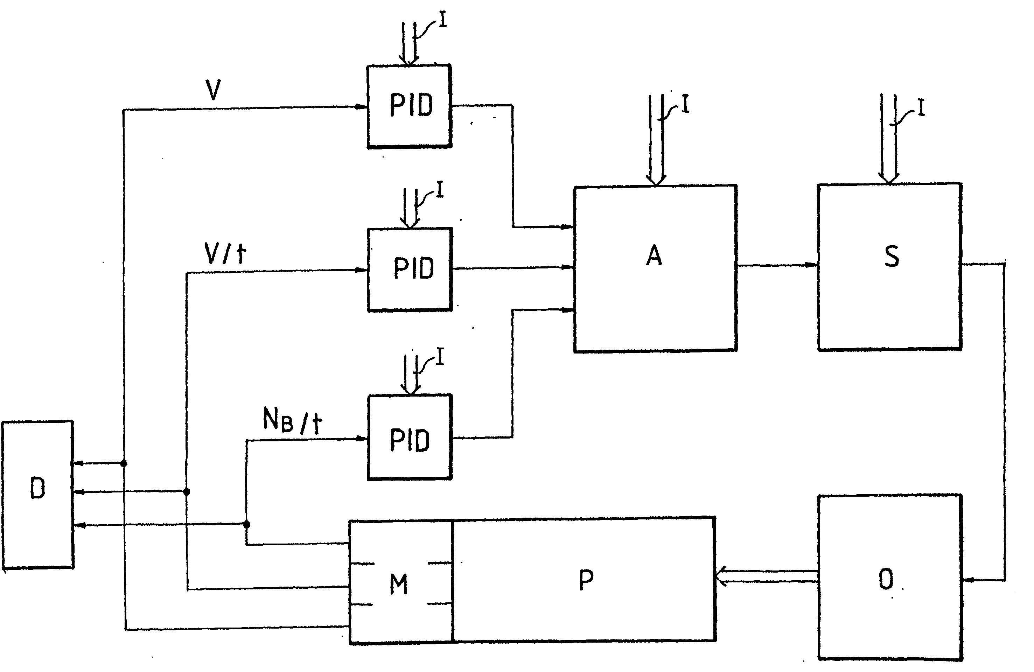

Weitere Merkmale, Einzelheiten, Vorteile und Wirkungen auf der Basis der Erfindung ergeben sich aus der nachfolgenden Beschreibung eines bevorzugten Ausführungswegs der Erfindung sowie aus der Zeichnung, die ein schematisches Blockschaltbild eines beispielhaften Test- und Analysesystems nach der Erfindung zeigt

Die Atmung einer Testperson P, insbesondere eines Vorschulkindes, wird mittels einer Meßsensorik M für das ein- und ausgeatmete Luftvolumen V, für den bidirektionalen Luft-Durchfluß V/t und für die Atemfrequenz N_{B}/t (Anzahl der Atmungen N_{B} pro Zeiteinheit t) erfaßt. Die entsprechenden Meßwerte V, V/t, N_{B}/t werden Filtern, gemäß gezeichnetem Beispiel PID-Regelgliedern jeweils zugeführt. Diese Filter bzw. Regelglieder führen eine Bewertung der genannten Meßgrößen durch, die von der Testperson P herrühren, die gleichsam die Regelstrecke in einem geschlossenen Regelkreis bildet.

Es ist zweckmäßig, die Meßergebnisse auch einem Display D parallel zuzuführen, wo auch sonstige Parameter in Echtzeit mit geeigneter Skalierung dargestellt werden können. Insbesondere können auf dem Display D auch Vorhersagewerte und Werte früherer Messungen eingeblendet werden.

Die Ausgänge der je einem Meßgrößentyp zugeordneten Filter PID sind einer gemeinsamen Recheneinheit A zugeführt. Diese kann beispielsweise als Summierer für die von den Filtern PID bewerteten Meßergebnisse ausgeführt sein. Ihr Ausgang steuert ein Speichermedium für Reiz-, Animations- oder sonstige Informationsmuster, die vor allem für Vorschulkinder geeignet sind, an. Die Ansteuerung kann beispielsweise über die Adreßeingänge des Speichermediums erfolgen, wenn dieses digital ausgeführt ist. Zusätzlich ist es zweckmäßig, wenn das Speichermedium mit einem weiteren Eingang I versehen ist, über den von extern Informationsmuster zugeführt oder auch innerhalb des Speichermediums gelöscht werden können. Der Inhalt des Speichermediums S, nämlich die Informationsmuster, werden einem Ausgabemedium O, beispielsweise einem Bildschirm, zugeführt, der von der Testperson visuell erfaßt wird. Zusätzlich kann die Ausgabeeinheit auch andere Schnittstellen akustischer Art (wie Lautsprecher) besitzen. Die aus dem Speichermedium S über die Ausgabeeinheit O an die Testperson P übermittelten Muster dienen dazu, deren Psyche und Nervensystem je nach Diagnosezweck zu animieren und zu stimulieren, wodurch die Art der Atmung der Testperson und damit die Atemmeßergebnisse V, V/t, N_{B}/t gezielt beeinflußt werden können. Das Ausgabemedium O stellt dabei gewissermaßen die Stelleinrichtung des Regelkreises dar, während die Filter PID mit Recheneinheit A und Speichermedium S den Regler bilden. Dieser kann über die Eingänge I in die Filter PID, Recheneinheit A und das Speichermedium S vor dem Regelvorgang parametrisiert oder während des Regelvorgangs an besondere, sich ändernde Umstände adaptiert werden.

Auf dem Ausgabemedium O läßt sich für Vorschulkinder ein Bild darstellen, das dem Vorschulkind aus seiner realen Umgebung bekannt ist. Das Kind kann das Bild interaktiv durch Atmen mit einem Mundstück oder einem Lungentachygraph verändern bzw. bearbeiten. Jedes Atmungsmanöver, sowohl Ein- als auch Ausatmen, verursacht eine vorbestimmte Bewegung des Bildelements. Die entsprechende Bildinformation ist im vorhinein im Speichermedium S abgelegt. Daraus können dem Kind Aufgaben bspw. in Form von Computer-Spielen präsentiert werden, wobei eine Belohnung oder Anerkennung erteilt wird, wenn das Ziel erreicht oder die Aufgabe gelöst ist Jedes im Speichermedium S abgelegte Spiel hat mehrere Schwierigkeitsgrade. Die Spiele sind so gestaltet, daß sie den gewünschten Lungen- oder Atmungstests dienen und gleichzeitig die Konzentrationsfähigkeit des Kindes berücksichtigen. Mit besonderem Vorteil wird jedes Spiel als eine "Kurzgeschichte" mit einem überraschenden Ende aufgeführt Die zugehörige Software ist zweckmäßig so geschrieben, daß sie mit gängigen Betriebssystemen und Krankenhaus-Datenbanken kompatibel ist.

## Patentansprüche

1. Vorrichtung zum Test und zur Analyse von Lungenfunktionen eines Patienten, nämlich einer Person, insbesondere eines Vorschulkindes, oder eines Tieres, mit
a) wenigstens einem, die Atmung des Patienten erfassenden Meßmittel (M), das als mit einem Mundstück versehener Volumenmesser für das Atemluft-Volumen (V) ausgebildet ist;
b) wenigstens einem Filter (PID), das dem Meßmittel (M) an dessen Ausgang nachgeschaltet ist;
c) einem Speicher (S) mit abgespeicherten Informationsmustern;
d) einem Medium (O) zur Ausgabe von Informationsmustern;
e) wobei der Musterspeicher (S) mit dem Ausgabemedium (O) zur Übertragung von Musterinformationen gekoppelt ist;
f) wobei die Filter (PID) und/oder die Recheneinheit (A) zur Weiterverarbeitung der Meßgröße(n) (V,V/t,N_{B}/t) in derartige Ansteuerungssignale für den Musterspeicher (S) ausgebildet sind, dass die von dem Musterspeicher (S) auszugebenden Informationen in das Ausgabemedium (O) in Abhängigkeit von der Atmung solchermaßen eingespeist werden, dass Informationselemente verändert werden, wobei auf jeweils im vorhinein abgelegte Informationen zurückgegriffen wird; und
g) wobei die abgespeicherten und auszugebenden Informationsmuster auf die Instinkte des Patienten einwirkende Stimulations- und/oder Animationsmuster sind;
**dadurch gekennzeichnet, dass**
h) zwei voneinander unterschiedliche Meßmittel (M) vorgesehen sind; und
i) jedem dieser Meßmittel (M) nachgeschaltete Filter (PID) ausgangsseitig über eine gemeinsame, zwischengeschaltete Recheneinheit (A) mit den Adreßeingängen des Musterspeichers (S) zu dessen Ansteuerung gekoppelt sind.

2. Vorrichtung nach Anspruch 1, **dadurch gekennzeichnet, dass** die Recheneinheit (A) als Summierglied ausgebildet ist.

3. Vorrichtung nach einem der vorhergehenden Ansprüche, **dadurch gekennzeichnet, dass** das oder die Meßmittel (M) als Volumenund/oder Durchflußmesser (V,V/t) für Atemluft, als Lungentachygraph und/oder als Zähler für die Frequenz (N_{B}/t) der Atmung ausgebildet sind.

4. Vorrichtung nach einem der vorhergehenden Ansprüche, **dadurch gekennzeichnet, dass** das oder die Filter (PID) als Bandpässe, insbesondere als PID-Regler, ausgebildet sind.

5. Vorrichtung nach einem der vorhergehenden Ansprüche, **dadurch gekennzeichnet, dass** das Ausgabemedium (O) als Bildschirm und/oder Lautsprecher ausgeführt ist.

## Claims

1. An apparatus for testing and analysis of the pulmonary functions of a patient, namely a person, in particular a preschool child, or an animal, having
a) at least one measuring means (M) detecting the patient's breathing, which means is in the form of a volume meter for the respiratory air volume (V), provided with a mouthpiece;
b) at least one filter (PID) which is connected to the output side of the measuring means (M);
c) a memory (S) with stored information patterns;
d) a medium (O) for the output of information patterns;
e) wherein the pattern memory (S) is coupled to the output medium (O) for the transmission of pattern information;
f) wherein the filters (PID) and/or the computing unit (A) are so designed for further processing the measured quantities (V,V/t,N_{B}/t) into such control signals for the pattern memory (S) that the data to be delivered by the pattern memory (S) are fed into the output medium (O) as a function of the respiration in such a way that data elements are varied, wherein previously stored data are retrieved in each case, and
g) wherein the information patterns stored and to be delivered are stimulation and/or animation patterns acting on the patient's instincts;
**characterised in that**
h) two measuring means (M) different from one another are provided, and
j) filters (PID) connected to the output side are coupled to each of these measuring means (M) via a common interconnected computing unit (A) with the address inputs of the pattern memory (S) for the activation thereof.

2. An apparatus according to Claim 1, **characterised in that** the computing unit (A) is in the form of a summing element.

3. An apparatus according to any one of the preceding Claims, **characterised in that** the measuring means (M) is/are in the form of a volume meter and/or flowmeter (V,V/t) for respiratory air, of a pulmonary tachygraph and/or as a counter for respiration frequency (N_{B}/t).

4. An apparatus according to any one of the preceding Claims, **characterised in that** the filter or filters (PID) are in the form of band passes, in particular of PID controllers.

5. An apparatus according to any one of the preceding Claims, **characterised in that** the output medium (O) is in the form of a display screen and/or loudspeaker.

## Revendications

1. Dispositif pour tester et pour analyser les fonctions pulmonaires d'un patient, à savoir d'une personne, notamment d'un enfant d'âge préscolaire, ou d'un animal, comportant
a) au moins un moyen de mesure (M), enregistrant la respiration du patient, lequel moyen de mesure est conçu sous forme d'un volumètre muni d'une embouchure, pour la mesure du volume d'air respiré (V);
b) au moins un filtre (PID), qui est connecté en aval du moyen de mesure (M), à la sortie de ce dernier;
c) une mémoire (S) renfermant des modèles d'informations mémorisés;
d) un support (O) pour la sortie de modèles d'informations;
e) la mémoire de modèle (S) étant alors couplée au support de sortie (O) pour la transmission d'informations d'un modèle;
f) les filtres (PID) et/ou l'unité de calcul (A) étant conçus pour transformer la (les) grandeur(s) de mesure (V, V/t, N_{B}/t) en des signaux d'excitation pour la mémoire de modèles (S) qui soient tels, que les informations appelées à être délivrées par la mémoire de modèles (S) sont admises dans le support de sortie (O), en fonction de la respiration, d'une manière telle que des éléments d'information soient modifiés, par recours à des informations respectives mises en dépôt par avance; et
g) les modèles d'informations mémorisés et appelés à être délivrés sont des modèles de stimulation et/ou d'animation agissant sur les instincts du patient;
**caractérisé en ce que**
h) deux moyens de mesure (M), différents l'un de l'autre, sont prévus; et
i) des filtres (PID), connectés en aval de chacun de ces moyens de mesure (M), sont couplés, par leur sortie, aux entrées d'adresse de la mémoire de modèles (S), en vue de son excitation, au travers d'une unité de calcul commune (A), intercalée.

2. Dispositif selon la revendication 1, **caractérisé en ce que** l'unité de calcul (A) est conçue sous forme d'un sommateur.

3. Dispositif selon l'une des revendications précédentes, **caractérisé en ce que** le ou les moyens de mesure (M) sont conçus sous forme de volumètre et/ou de débitmètre (V, V/t) pour l'air respiré, sous forme de tachygraphe pulmonaire et/ou sous forme de compteur pour la fréquence (N_{B}/t) de la respiration.

4. Dispositif selon l'une des revendications précédentes, **caractérisé en ce que** le ou les filtres (PID) sont conçus sous forme de passe-bande, en particulier sous forme de régulateurs (PID).

5. Dispositif selon l'une des revendications précédentes, **caractérisé en ce que** le support de sortie (O) est réalisé sous forme d'écran et/ou de haut-parleur.
